# EUROPEAN PATENT SPECIFICATION

(11) **EP 4 461 718 B1**
(45) Date of publication and mention of the grant of the patent: **31.12.2025**
(21) Application number: 23210889.4
(22) Date of filing: 20.11.2023
(51) Int. Cl.: C07C 2/66, C07C 2/68, C07C 2/70, C07C 5/333, C07C 7/163, C07C 7/167, C07C 11/02, C07C 15/107, C10G 3/00, C10G 29/20, C10G 69/04, C10G 69/12

(54) **PROCESS FOR PRODUCING RENEWABLE ALKYLBENZENE PRODUCTS**
VERFAHREN ZUR HERSTELLUNG VON ERNEUERBAREN ALKYLBENZOLPRODUKTEN
PROCÉDÉ DE PRODUCTION DE PRODUITS D'ALKYLBENZÈNE RENOUVELABLES

(30) Priority: 30.05.2023 US 202363504879 P
(43) Date of publication of application: 13.11.2024
(73) Proprietor: UOP LLC, Rosemont, Illinois 60018 (US)
(72) Inventor: DO, Phuong T.M., Charlotte, 28202 (US); DU, Hai, Charlotte, 28202 (US); FICHTL, Geofffrey W., Charlotte, 28202 (US); WEXLER, James T., Charlotte, 28202 (US); JERORO, Eseoghene, Charlotte, 28202 (US); WHITCHURCH, Patrick C., Charlotte, 28202 (US)
(74) Representative: Boult Wade Tennant LLP

(56) References cited:
- US-A1- 2013 317 267
- US-A1- 2015 361 012

## Description

### BACKGROUND

Linear alkylbenzenes are organic compounds with the formula C₆H₅CₙH₂ₙ₊₁. While the alkyl carbon number, "n" can have any practical value, detergent manufacturers desire that alkylbenzenes have alkyl carbon number in the range of 9 to 16 and preferably in the range of 9 to 14. These specific ranges are often required when the alkylbenzenes are used as intermediates in the production of surfactants for detergents. The alkyl carbon number in the range of 9 to 14 falls in line with the specifications of the detergents industry.

Because the surfactants created from alkylbenzenes are biodegradable, the production of alkylbenzenes has grown rapidly since their initial uses in detergent production in the 1960s. The linearity of the paraffin chain in the alkylbenzenes is key to the material's biodegradability and effectiveness as a detergent. A major factor in the final linearity of the alkylbenzenes is the linearity of the paraffin component.

While detergents made utilizing alkylbenzene-based surfactants are biodegradable, previous processes for creating alkylbenzenes are not based on renewable sources. Specifically, alkylbenzenes are currently produced from kerosene refined from crude extracted from the earth. Due to the growing environmental prejudice against fossil fuel extraction and economic concerns over exhausting fossil fuel deposits, there may be support for using an alternate source for biodegradable surfactants in detergents and in other industries.

Accordingly, it is desirable to provide linear alkylbenzenes with a high degree of linearity that are made from biorenewable sources instead of being extracted from the earth. Further, it is desirable to provide renewable linear alkylbenzenes from easily processed triglycerides and fatty acids from vegetable, animal, nut, and/or seed oils. Palm kernel oil, coconut oil and babassu oil have a composition that is high in the desirable range of C9-C14 n-paraffins that aligns with the alkyl carbon number range desired of the detergent industry. Such renewable sources also have a high amount of nC16 to nC18 feeds, and it is desirable to convert those feeds to nC9 to nC14 feeds with a high per-pass yield. These nC9 to nC14 intermediate products are useful in eventually making linear alkylbenzene types of detergents through additional process steps. It is further desirable that the resulting nC9 to nC14 paraffins are linear products with a minimum of branched isomer products.

US 2013/317267 A1 relates to methods for co-production of alkylbenzene and biofuel from natural oils using hydrocracking.

US 2015/361012 A1 relates to methods for producing linear alkylbenzenes, paraffins, and olefins from natural oils and kerosene.

### BRIEF DESCRIPTION OF THE DRAWINGS

FIG. 1 is a schematic view of one embodiment of a process for producing alkylbenzenes from triglycerides according to the present invention.
Fig. 2 is a plot of the mass-% normal paraffins versus deoxygenation temperature in accordance with Example 2.

### DETAILED DESCRIPTION

The invention is in accordance with the appended claims. The present invention relates to a process for producing alkylbenzenes from triglycerides, in particular triglycerides producing 60 wt.% or more of normal paraffins having less than 16 carbon atoms after hydrogenation. Some of these triglycerides also produce a substantial amount of normal paraffins having 16 to 24 carbon atoms, e.g., 20 to 30%, after dehydrogenation. These paraffins are longer than desired for the production of detergent products.

In some embodiments, the amount of normal paraffins having 16 carbon atoms after dihydrogenation is less than 20%, or less than 15%, or less than 10%.

The triglycerides come from natural oils. Natural oils are not based on kerosene or other fossil fuels. Natural oils include those derived from plant or algal material or animal fats, nut, and/or seed oils, and triglyceride-containing oils, and are often referred to as renewable oils. Natural oils typically comprise triglycerides, free fatty acids, or combinations thereof. Natural oils include, but are not limited to, Arachis oil (peanut oil; groundnut oil), Babassu oil, Coconut oil, Cottonseed oil, Grapeseed oil, Maize oil (corn oil), Mustard seed oil, Palm kernel oil, Palm oil, Palm olein (the liquid fraction derived from the fractionation of palm oil), Palm stearin (the high-melting fraction derived from the fractionation of palm oil), Rapeseed oil, Rapeseed oil - low erucic acid (low erucic acid turnip rape oil; low erucic acid colza oil; canola oil), Safflowerseed oil (safflower oil; carthamus oil; kurdee oil), Safflowerseed oil - high oleic acid (high oleic acid safflower oil; high oleic acid carthamus oil; high oleic acid kurdee oil), Sesameseed oil (sesame oil; gingelly oil; benne oil; ben oil; till oil; tillie oil) , Soya bean oil (soybean oil), Sunflowerseed oil (sunflower oil), and Sunflowerseed oil - high oleic acid (high oleic acid sunflower oil).

In some embodiments, the triglyceride produces no less than 15 wt.% of normal paraffins having 12 or 14 carbon atoms after deoxygenation. In some embodiments, the triglyceride produces no less than 10 wt.% of normal paraffins having 12 carbon atoms after deoxygenation.

The process for making alkylbenzenes from triglycerides according to the present invention involves the deoxygenation of the triglycerides to form paraffins. The paraffins are separated (by fractionation, distillation, and the like) into a C9 to C14 stream comprising C9 to C14 paraffins and a C14+ stream comprising C14+ (i.e., containing carbon chains from C15 to C28) paraffins. The C14+ stream is sent to a separate linear selective cracking unit to crack the C14+ paraffins; the cracked paraffins are fractionated into a first stream comprising the C9 to C14 normal and lightly branched paraffins and a second stream comprising isoparaffins. Contaminants, including but not limited to, comprise sulfur compounds, or nitrogen compounds, or phosphorous compounds, or oxygenates, or aromatics or combinations thereof, are removed from the C9 to C14 stream and the first stream. The decontaminated stream is dehydrogenated to form olefins, di-olefins, and aromatics. The di-olefins are selectively hydrogenated to form additional olefins, and the aromatics are separated and removed forming an aromatics stream comprising the aromatics and a mono-olefin stream comprising the mono-olefins. Benzene is alkylated with the olefins, and the alkylation effluent comprises alkylbenzenes and benzene. The alkylbenzenes are then isolated.

The linear selective cracking step will be further described. The linear selective cracking takes place in a separate unit, rather than in the bottom bed of a first stage hydrocracking reactor because sulfur and nitrogen contaminants from the first stage can poison a metal-based hydrocracking catalyst. The C14+ paraffins are selectively cracked over the C9 to C14 due to higher absorption energy.

Selection of particular metal catalysts, including noble metals (such as ruthenium and platinum), and nickel can produce a much higher yield of normal paraffins with 9-14 carbons than previous processes. Suitable catalysts include, but are not limited to, Ru/ZrO₂, a Pt-Al₂O₃, Ni-alumina, or a NiOₓ/clay. With these catalysts, the C14+ stream can generate linear cracking products without significant amounts of branched isomer production.

Of the preferred catalysts, the Ru catalyst exhibits much higher activity and per-pass nC9 to nC14 yield than the other catalysts. Under the optimized reaction conditions, it also produces very small amounts of methane and isomerized product. This has been found to be the best catalyst for such chemical transformation process. The Pt-Al₂O₃ catalyst can produce even lower methane yield than the Ru based catalyst with slightly less linear product yield.

To limit catalyst deactivation, the feed is treated to remove sulfur, chloride and metal contamination before hydrodeoxygenation. Otherwise, sulfur, chloride and metals accumulate on the catalyst, and this leads to deactivation. A high temperature hydrogen treatment was shown to recover some of the lost activity. The degree of hydrodeoxygenation can affect the selectivity to each of the normal paraffins in the 9 to 14 carbon range. A large degree of hydrodeoxygenation can bias the hydrodeoxygenated composition largely in favor of normal dodecane and normal decane to the detriment of normal undecane and normal tridecane. A small degree of hydrodeoxygenation can bias the hydrodeoxygenated composition in favor of normal undecane and normal tridecane to the detriment of normal dodecane and normal decane.

The hydrodeoxygenation reactor temperatures are kept low, less than 343°C (650°F) for typical biorenewable feedstocks and less than 304°C (580°F) for feedstocks with higher free fatty acid (FFA) concentration to avoid polymerization of olefins found in FFA. Generally, hydrodeoxygenation reactor pressure of 700 kPa (100 psig) to 21 MPa (3000 psig) are suitable.

The overall process will now be described.

The linearity of the alkylbenzene product is mostly dependent on the linearity of the paraffins used to alkylate the benzene. It is a common rule of thumb by those skilled in the art that the linearity of a paraffin feed drops by 5-7 mass % after dehydrogenation and alkylation. Therefore, paraffin with 97 mass % linearity (or alternatively 3 mass % isoparaffin) would result in an alkylbenzene product with linearity around 90-92 mass %. This sets the requirement for paraffin linearity 5-7 mass % higher than the specification for the alkylbenzene product. Typically the linearity of the paraffin product is measured by UOP 621, UOP411, or UOP732 standard test method available from ASTM. Linear alkylbenzenes may be analyzed using ASTM Standard Test Method D4337.

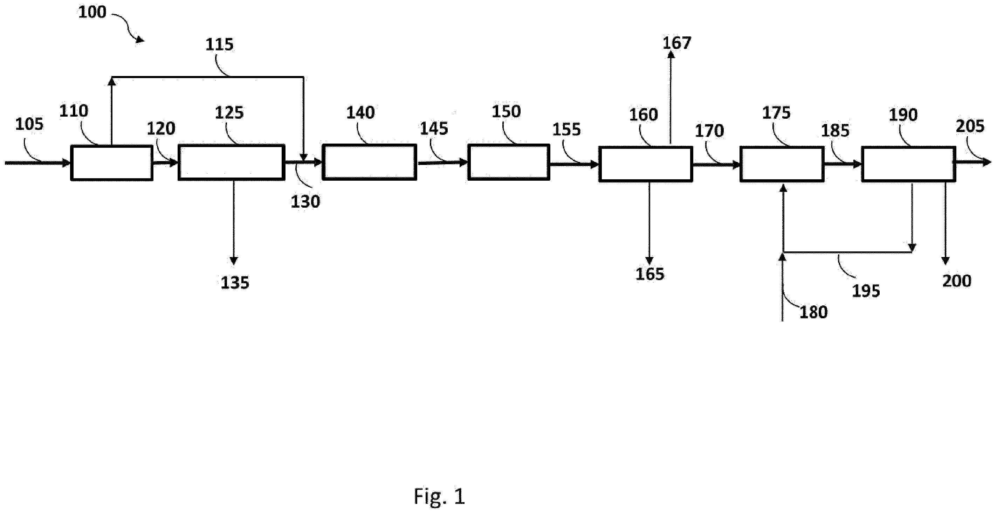

In Figure 1, an exemplary system 100 for producing an alkylbenzene product from a specific triglyceride feed is illustrated.

In the illustrated embodiment, the selected triglyceride feed 105 is delivered to a deoxygenation unit 110 which also receives a hydrogen feed (not shown). In the deoxygenation unit 110, the fatty acids in the selected triglyceride feed 105 are deoxygenated and converted into normal paraffins. Structurally, triglycerides are formed by three, typically different, fatty acid molecules that are bonded together with a glycerol bridge. The glycerol molecule includes three hydroxyl groups (HO--) and each fatty acid molecule has a carboxyl group (COOH). In triglycerides, the hydroxyl groups of the glycerol join the carboxyl groups of the fatty acids to form ester bonds. Therefore, during deoxygenation, the fatty acids are freed from the triglyceride structure and are converted into normal paraffins. The glycerol is converted into propane, and the oxygen in the hydroxyl and carboxyl groups is converted into water, carbon dioxide, or carbon monoxide. The deoxygenation reaction for fatty acids and triglycerides are respectively illustrated as:

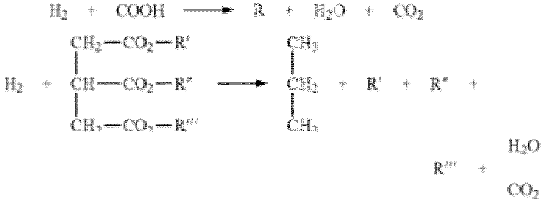

During the deoxygenation reaction, the length of a paraffin chain Rⁿ created will vary by a value of one depending on the exact reaction pathway. It is understood that deoxygenation includes at least one of hydrodeoxygenation, decarboxylation, and decarbonylation, or any combination thereof. For instance, if carbon dioxide is formed, then the chain will have one fewer carbon than the fatty acid source. If water is formed, then the chain will match the length of the fatty acid source.

Operating conditions for the deoxygenating unit include pressures in the range of from 1724 to 5516 kPa (250 to 800 psig) and temperatures in the range of from 274°C to 371°C (525°F to 700°F) in one embodiment, from 274°C to 338°C (525°F to 640°F) in another embodiment and from 274°C to 310°C (525°F to 590°F) in another embodiment. Catalysts may include those containing one or more of Ni, Mo, Co, P, such as Ni--Mo, Ni-Mo--P, Ni--Co--Mo, or Co--Mo, on alumina, silica, titania, zirconia, and mixtures thereof. The hydrogen to hydrocarbon mole ratios include from 267.16 to 1781.08 (1500 to 10,000), from 712.43 to 1602.97 (4000 to 9000), and from 890.54 to 1424.86 (5000-8000) m³ per m³ of feedstock (standard cubic feet per barrel of feedstock (scf/B)). The space velocities include 0.2-3.0 hr⁻¹ LHSV. Conditions are selected to minimize cracking or isomerizing the paraffins.

The deoxygenated product containing normal paraffins, water, carbon dioxide, carbon monoxide, and propane is fractionated into a C9 to C14 stream 115 and a C14+ stream 120. The separation may be performed in a multi-stage fractionation unit, distillation system or similar known apparatus. In any event, the separator removes the water, carbon dioxide, carbon monoxide, and propane from the deoxygenated product. A naphtha stream of paraffins with carbon chain lengths of C₅ to C₉ (not shown) may also be formed.

The C14+ stream 120 is sent to the linear selective cracking unit 125 where it is selectively cracked to form a first stream 130 comprising normal or lightly branched C9 to C14 paraffins and a second stream 135 comprising isoparaffins, as described above.

The C9 to C14 stream 115 from the deoxygenation unit 110 and the first stream 130 from the linear selective cracking unit 125 are sent to a decontamination unit 140. The decontamination unit 140 removes contaminants in an adsorption system from the C9 to C14 paraffins in the C9 to C14 stream 115 and the first stream 130. The contaminants include, but are not limited to, sulfur compounds, or nitrogen compounds, or phosphorous compounds, or oxygenates, or aromatics or combinations thereof.

The decontaminated stream 145 is sent to a dehydrogenation unit 150 where hydrogen is removed to produce a dehydrogenated stream 155 comprising mono-olefins, di-olefins, and aromatics. In the dehydrogenation unit 150, the paraffins are dehydrogenated into mono-olefins of the same carbon numbers as the paraffins. Typically, dehydrogenation occurs through known catalytic processes, such as the commercially popular Pacol process. Di-olefins (i.e., dienes) and aromatics are also produced as an undesired result of the dehydrogenation reactions as expressed in the following equations:
Mono-olefin formation: CₓHa₂ₓ₊₂→ CₓH₂ₓ + H₂
Di-olefin formation: CₓH₂ₓ - CₓH₂ₓ₋₂ + H₂
Aromatic formation: CₓH₂ₓ₋₂ - CₓH₂ₓ₋₆ + 2H₂

Operating conditions for the dehydrogenation unit 150 include space velocities from 5 to 50 LHSV and from 20 to 32 LHSV; pressures from 1 kPa (g) to 1013 kPa (g) (0.1 psig to 150 psig); temperatures from 400°C to 500°C and from 440°C to 490°C, and hydrogen to hydrocarbon mole ratios from 1-12 and from 3-7. An example of a suitable catalyst is a Pt on alumina catalyst where platinum is attenuated with an attenuator metal. Another suitable catalyst is described in U.S. Pat. No. 6,177,381. The dehydrogenation unit 150 may be operated dry or with water injection up to 2000 mass-ppm water. Hydrogen can be recycled to the deoxygenation unit upstream.

The dehydrogenated stream 155 is sent to a selective hydrogenation unit 160, such as a DeFine reactor, where at least a portion of the di-olefins are hydrogenated to form additional mono-olefins. As a result, the mono-olefin stream 170 has an increased mono-olefin concentration compared to the dehydrogenated stream 155. The aromatics are separated and removed as aromatics stream 165. A light end stream 167 containing any lights, such as butane, propane, ethane and methane, that resulted from cracking or other reactions during upstream processing can also removed.

The mono-olefin stream 170 comprising mono-olefins is sent to the alkylation unit 175 along with a benzene stream 180. The benzene is alkylated with the mono-olefins to form alkylbenzene. The alkylation unit 175 contains a catalyst, such as a solid acid catalyst, that supports alkylation of the benzene with the mono-olefins. Fluorinated silica-alumina, hydrogen fluoride (HF), aluminum chloride (AlCl₃), zeolitic, and ionic liquid catalysts are examples of major catalysts in commercial use for the alkylation of benzene with linear mono-olefins and may be used in the alkylation unit 175. As a result of alkylation, alkylbenzene, typically called linear alkylbenzene (LAB), is formed according to the reaction:

C₆H₆ ⁺ CₓH₂ₓ → C₆H₅C₆H₂ₓ₊₁

Suitable operating conditions for the alkylation unit 175 include space velocities from 1 to 10 LHSV, pressures to maintain liquid phase operation, such as 2068 kPa (g) to 4137 kPa (g) (300 psig to 600 psig), temperatures in the range of from 80°C to 180°C and 120°C to 170°C, benzene to olefin mole ratios of 3 to 40 and 8 to 35.

Surplus amounts of benzene are supplied to the alkylation unit 175 to achieve high degree of desired alkylation. Therefore, the alkylation effluent 185 exiting the alkylation unit 175 contains alkylbenzene and unreacted benzene. Further the alkylation effluent 185 may also include some unreacted paraffins. The alkylation effluent 185 is passed to a benzene separation unit 190, such as a fractionation column, for separating the unreacted benzene and paraffins from the alkylation effluent 185. The unreacted benzene exits the benzene separation unit 190 in a benzene recycle stream 195 that may be sent back into the alkylation unit 175 to maintain the desired benzene/olefin ratio (e.g., 1-50) to reduce the volume of fresh benzene needed. The fresh benzene requirement (i.e., the net benzene) is determined by the net olefin to the alkylation unit. A paraffin stream 200 can also be separated out and recycled to the dehydrogenation unit 150.

As a result of the post-alkylation separation processes, the linear alkylbenzene product 205 is isolated. It is noted that such separation processes are not necessary in all embodiments in order to isolate the linear alkylbenzene product 205.

The linear alkylbenzene product 205 is a linear alkylbenzene product comprising: alkylbenzenes having the formula C₆H₅CₙH₂ₙ₊₁ wherein n is from 9 to 14. In some embodiments, at least 80 mass % of the alkylbenzenes have linear alkyl groups, or at least 90 mass %.

The linear alkylbenzene may be sulfonated to provide a linear alkylbenzene sulfonate product comprising: alkylbenzene sulfonate compounds having the formula CₙH₂ₙ₊₁C₆H₄SO₃H wherein n is from 10 to 14, or wherein n is from 11 to 13.

As used herein, the term "separator" means a vessel which has an inlet and at least an overhead vapor outlet and a bottoms liquid outlet and may also have an aqueous stream outlet from a boot. A flash drum is a type of separator which may be in downstream communication with a separator which may be operated at higher pressure. The term "communication" means that fluid flow is operatively permitted between enumerated components, which may be characterized as "fluid communication". The term "downstream communication" means that at least a portion of fluid flowing to the subject in downstream communication may operatively flow from the object with which it fluidly communicates.

The term "column" means a distillation column or columns for separating one or more components of different volatilities. Unless otherwise indicated, each column includes a condenser on an overhead of the column to condense and reflux a portion of an overhead stream back to the top of the column and a reboiler at a bottom of the column to vaporize and send a portion of a bottoms stream back to the bottom of the column. Feeds to the columns may be preheated. The top pressure is the pressure of the overhead vapor at the vapor outlet of the column. The bottom temperature is the liquid bottom outlet temperature. Unless indicated otherwise, overhead lines and bottoms lines refer to the net lines from the column downstream of any reflux or reboil take-off to the column. Stripper columns may omit a reboiler at a bottom of the column and instead provide heating requirements and separation impetus from a fluidized inert media such as steam.

As used herein, the term "a component-rich stream" or "a component stream" means that the stream coming out of a vessel has a greater concentration of the component than the feed to the vessel. As used herein, the term "a component-lean stream" means that the lean stream coming out of a vessel has a smaller concentration of the component than the feed to the vessel.

### EXAMPLES

### Example 1

A coconut oil feed was deoxygenated, to form paraffins, dehydrogenated to form mono-olefins, and benzene was alkylated with the mono-olefins to form an alkylbenzene product with a modern carbon content of 62 mass % modern carbon as determined by ASTM D6866 as compared to a theoretical modern carbon content of 66.4 mass %, a bromine number of 1 g Br/per gram sample as determined by UOP standard test method 304, and a linearity of 92 mass %.

### Example 2

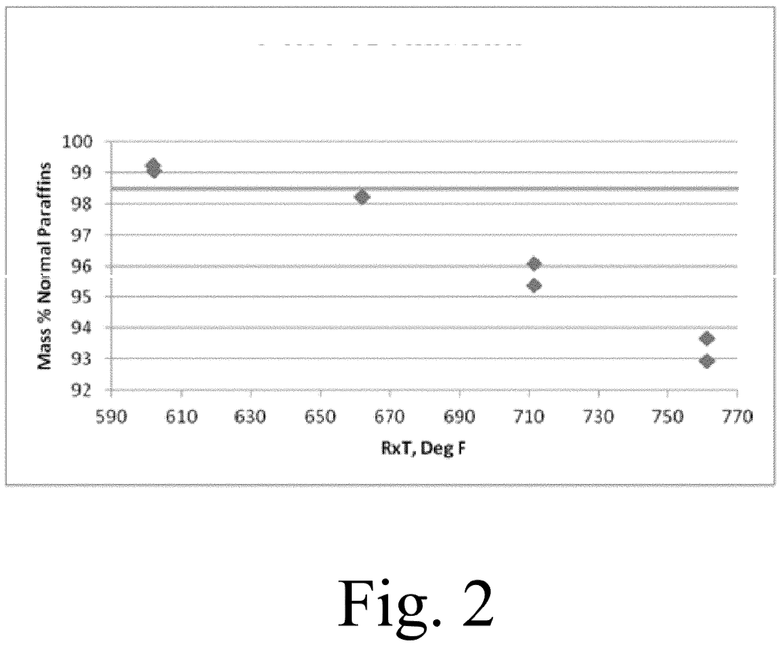

An oil was deoxygenated using a catalyst at a pressure of 3301 kPa (480 psig), H to bio-oil ratio of 1282,37 m³ /m³ (7200 scf/B) and a LHSV of 1 hr'. During operation, the deoxygenation reaction temperature was increased in steps from 315°C 315∘AC.(600°F) to 34.9°C (660°F) and then to 377°C (710°F) and 404°C (760°F) to monitor the response of linearity in the final product to reaction temperature. The results are shown in Fig. 2 which is a plot of the concentration in mass % of normal C10-C 13 paraffins versus reaction temperature. Fig. 2 clearly demonstrates that as the deoxygenation reaction temperature is increased, the concentration of linear paraffins decreases. Controlling the temperature to less than 404°C (760°F) resulted in greater than 92 mass percent linear paraffins.

Note: Examples 1 and 2 were previously included in US Patent No. 9,079,814 as Examples 3 and 4.

In the foregoing, all temperatures are set forth in degrees Celsius and, all parts and percentages are by weight, unless otherwise indicated.

## Claims

1. A method for production of a linear alkylbenzene product derived from a triglyceride comprising:
deoxygenating a triglyceride (105) which produces 60% or more of normal paraffins having less than 16 carbon atoms after deoxygenation to form a paraffin stream comprising 60% or more of normal paraffins having less than 16 carbon atoms;
fractionating the paraffin stream to form a C9 to C14 stream (115) comprising C9 to C14 paraffins and a C14+ stream (120) comprising C14+ paraffins;
linear selective cracking the C14+ stream (120) in a separate linear selective cracking unit (125) under linear selective cracking conditions in the presence of a linear selective cracking catalyst to form a first stream (130) comprising normal or lightly branched C9 to C14 paraffins and a second stream (135) comprising isoparaffins;
removing contaminants from the C9 to C14 stream (115) and the first stream (130) to form a decontaminated stream (145) wherein the contaminates comprise sulfur compounds, or nitrogen compounds, or phosphorous compounds, or oxygenates, or aromatics or combinations thereof;
dehydrogenating the decontaminated stream (145) to provide a dehydrogenated stream (155) comprising mono-olefins, di-olefins, and aromatics;
selectively hydrogenating the di-olefins in the dehydrogenated stream (155) to form additional mono-olefins, and separating and removing the aromatics from the mono-olefins to form an aromatics stream (165) comprising the aromatics and a mono-olefins stream (170) comprising the mono-olefins;
alkylating benzene (180) with the mono-olefins under alkylation conditions to provide an alkylation effluent (185) comprising alkylbenzenes and benzene; and
isolating the alkylbenzenes to provide the alkylbenzene product (205) derived from the triglyceride;
wherein deoxygenating the triglyceride (105) is done in a deoxygenating unit, and wherein the operating conditions for the deoxygenating unit include pressures in the range of 1724 to 5516 kPa (250 to 800 psig), temperatures in the range of from 274°C to 371°C (525°F to 700°F), hydrogen to hydrocarbon mole ratios of from 267.16 to 1781.08 m³ per m³ of feedstock (1500 to 10,000 standard cubic feet per barrel of feedstock), and liquid hourly space velocities (LHSV) of 0.2 to 3.0 hr⁻¹;
wherein the triglyceride (105) comes from natural oils, wherein natural oils are not based on kerosene or other fossil fuels, and natural oils include those derived from plant or algal material or animal fats, nut, and/or seed oils, and triglyceride-containing oils.

2. The method of claim 1 wherein the C9 to C14 stream (115) and the first stream (130) are combined before removing the contaminants from the C9 to C14 stream (115) and the first stream (130) to form the decontaminated stream (145).

3. The method of any one of claims 1-2 wherein the linear selective cracking catalyst comprises ruthenium, platinum, and nickel supported catalyst or mixtures thereof.

4. The method of any one of claims 1-2 wherein the linear selective cracking conditions comprise a temperature in a range of 290 °C to 455°C, or a pressure in a range of 2.8 MPa to 17.5 MPa, or combinations thereof.

5. The method of any one of claims 1-2 wherein the alkylbenzene product (205) comprises alkylbenzenes having C9 to C14 chains.

6. The method of any one of claims 1-2 wherein the alkylbenzene product (205) comprises alkylbenzenes having C10 to C13 chains.

7. The method of any one of claims 1-2 wherein the C14+ stream (120) comprises C16 to C18 paraffins.

## Patentansprüche

1. Verfahren zur Produktion eines linearen Alkylbenzolprodukts, das aus einem Triglycerid abgeleitet ist, umfassend:
Deoxygenieren eines Triglycerids (105), das 60 % oder mehr normale Paraffine produziert, die weniger als 16 Kohlenstoffatome nach der Deoxygenierung aufweisen, um einen Paraffinstrom auszubilden, der 60 % oder mehr normale Paraffine, die weniger als 16 Kohlenstoffatome aufweisen, umfasst;
Fraktionieren des Paraffinstroms, um einen C9-bis-C14-Strom (115), der C9-bis-C14-Paraffine umfasst, und einen C14+-Strom (120), der C14+-Paraffine umfasst, auszubilden;
lineares selektives Cracken des C14+-Stroms (120) in einer separaten lineares-selektives-Cracking-Einheit (125) unter lineares-selektives Cracking-Bedingungen in Anwesenheit eines lineares-selektives-Cracking-Katalysators, um einen ersten Strom (130), der normale oder leicht verzweigte C9-bis-C14-Paraffine umfasst, und einen zweiten Strom (135), der Isoparaffine umfasst, auszubilden;
Entfernen von Kontaminationsstoffen aus dem C9-bis-C14-Strom (115) und dem ersten Strom (130), um einen dekontaminierten Strom (145) auszubilden, wobei die Kontaminationsstoffe Schwefelverbindungen oder Stickstoffverbindungen oder Phosphorverbindungen oder Oxygenate oder Aromate oder Kombinationen derselben umfassen;
Dehydrieren des dekontaminierten Stroms (145), um einen dehydrierten Strom (155) bereitzustellen, der Mono-Olefine, Di-Olefine und Aromate umfasst;
selektives Hydrieren der Di-Olefine in dem dehydrierten Strom (155), um zusätzliche Mono-Olefine auszubilden, und Trennen und Beseitigen der Aromaten von den Mono-Olefinen, um einen Aromatenstrom (165), der die Aromaten umfasst, und einen Mono-Olefinen-Strom (170), der die Mono-Olefine umfasst, auszubilden;
Alkylieren von Benzol (180) mit den Mono-Olefinen unter Alkylierungsbedingungen, um einen Alkylierungsabstrom (185) bereitzustellen, umfassend Alkylbenzole und Benzol; und
Isolieren der Alkylbenzole, um das aus dem Triglycerid abgeleitete Alkylbenzolprodukt (205) bereitzustellen;
wobei das Deoxygenieren des Triglycerids (105) in einer Deoxygenierungseinheit erfolgt, und wobei die Betriebsbedingungen für die Deoxygenierungseinheit Drücke in dem Bereich von 1724 bis 5516 kPa (250 bis 800 psig), Temperaturen in dem Bereich von 274 °C bis 371 °C (525 °F bis 700 °F), Wasserstoff-Kohlenwasserstoff-Molverhältnisse von 267,16 bis 1781,08 m³ pro m³ Ausgangsmaterial (1500 bis 10.000 Standardquadratfuß pro Barrel Ausgangsmaterial) und stündliche Flüssigkeitsraumgeschwindigkeiten (LHSV) von 0,2 bis 3,0 h⁻¹ beinhalten;
wobei das Triglycerid (105) aus natürlichen Ölen stammt, wobei natürliche Öle nicht auf Kerosin oder anderen fossilen Brennstoffen basieren, und natürlich Öle solche Öle, die aus Pflanzen- oder Algenmaterial oder tierischen Fetten abgeleitet sind, Nuss-und/oder Samenöle sowie Triglycerid-haltige Öle beinhalten.

2. Verfahren nach Anspruch 1, wobei der C9-bis-C14-Strom (115) und der erste Strom (130) vor dem Beseitigen der Kontaminationsstoffe aus dem C9-bis-C14-Strom (115) und dem ersten Strom (130), um den dekontaminierten Strom (145) auszubilden, kombiniert werden.

3. Verfahren nach einem der Ansprüche 1 bis 2, wobei der lineares-selektives-Cracking-Katalysator einen Ruthenium-, Platin- und Nickel-geträgerten Katalysator oder Mischungen derselben umfasst.

4. Verfahren nach einem der Ansprüche 1 bis 2, wobei die lineares-selektives-Cracking-Bedingungen Folgendes umfassen: eine Temperatur in einem Bereich von 290 °C bis 455 °C oder einen Druck in einem Bereich von 2,8 MPa bis 17,5 MPa oder Kombinationen derselben.

5. Verfahren nach einem der Ansprüche 1 bis 2, wobei das Alkylbenzolprodukt (205) Alkylbenzole umfasst, die C9-bis-C14-Ketten aufweisen.

6. Verfahren nach einem der Ansprüche 1 bis 2, wobei das Alkylbenzolprodukt (205) Alkylbenzole umfasst, die C10-bis-C13-Ketten aufweisen.

7. Verfahren nach einem der Ansprüche 1 bis 2, wobei der C14+-Strom (120) C16-bis-C18-Paraffine umfasst.

## Revendications

1. Procédé de production d'un produit alkylbenzène linéaire dérivé d'un triglycéride comprenant :
la désoxygénation d'un triglycéride (105) qui produit 60 % ou plus de paraffines normales ayant moins de 16 atomes de carbone après désoxygénation pour former un flux de paraffine comprenant 60 % ou plus de paraffines normales ayant moins de 16 atomes de carbone ;
le fractionnement du flux de paraffines pour former un flux de C9 à C14 (115) comprenant des paraffines C9 à C14 et un flux de C14+ (120) comprenant des paraffines C14+ ;
le craquage sélectif linéaire du flux C14+ (120) dans une unité distincte de craquage sélectif linéaire (125) dans des conditions de craquage sélectif linéaire en présence d'un catalyseur de craquage sélectif linéaire pour former un premier flux (130) comprenant des paraffines C9 à C14 normales ou légèrement ramifiées et un second flux (135) comprenant des isoparaffines ;
l'élimination des contaminants du flux C9 à C14 (115) et du premier flux (130) pour former un flux décontaminé (145) dans lequel les contaminants comprennent des composés soufrés, ou des composés azotés, ou des composés phosphorés, ou oxygénés, ou aromatiques ou des combinaisons de ceux-ci ;
la déshydrogénation du flux décontaminé (145) pour fournir un flux déshydrogéné (155) comprenant des mono-oléfines, des di-oléfines et des aromatiques ;
l'hydrogénation sélective des di-oléfines dans le flux déshydrogéné (155) pour former des mono-oléfines supplémentaires, et la séparation et l'élimination des aromatiques des mono-oléfines pour former un flux d'aromatiques (165) comprenant les aromatiques et un flux de mono-oléfines (170) comprenant les mono-oléfines ;
l'alkylation du benzène (180) avec les mono-oléfines dans des conditions d'alkylation pour fournir un effluent d'alkylation (185) comprenant des alkylbenzènes et du benzène ; et
l'isolation des alkylbenzènes pour fournir le produit alkylbenzène (205) dérivé du triglycéride ;
dans lequel la désoxygénation du triglycéride (105) est réalisée dans une unité de désoxygénation, et dans lequel les conditions de fonctionnement pour l'unité de désoxygénation incluent des pressions dans la plage de 1724 à 5516 kPa (250 à 800 psig), des températures dans la plage de 274 °C à 371 °C (525 °F à 700 °F), des rapports molaires hydrogène sur hydrocarbure de 267,16 à 1781,08 m³ par m³ de matière première (1500 à 10 000 pieds cubes standard par baril de matière première), et des vitesses spatiales horaires de liquide (LHSV) de 0,2 à 3,0 h⁻¹ ;
dans lequel le triglycéride (105) provient d'huiles naturelles, dans lequel les huiles naturelles ne sont pas basées sur du kérosène ou d'autres combustibles fossiles, et les huiles naturelles incluent celles dérivées de matière végétale ou algale ou de graisses animales, les huiles de noix et/ou de graines et les huiles contenant des triglycérides.

2. Procédé selon la revendication 1 dans lequel le flux C9 à C14 (115) et le premier flux (130) sont combinés avant d'éliminer les contaminants du flux C9 à C14 (115) et du premier flux (130) pour former le flux décontaminé (145).

3. Procédé selon l'une quelconque des revendications 1 et 2, dans lequel le catalyseur de craquage sélectif linéaire comprend un catalyseur à base de ruthénium, de platine et de nickel ou des mélanges de ceux-ci.

4. Procédé selon l'une quelconque des revendications 1 et 2, dans lequel les conditions de craquage sélectif linéaire comprennent une température dans une plage de 290 °C à 455 °C, ou une pression dans une plage de 2,8 MPa à 17,5 MPa, ou des combinaisons de celles-ci.

5. Procédé selon l'une quelconque des revendications 1 et 2, dans lequel le produit alkylbenzène (205) comprend des alkylbenzènes ayant des chaînes en C9 à C14.

6. Procédé selon l'une quelconque des revendications 1 et 2, dans lequel le produit alkylbenzène (205) comprend des alkylbenzènes ayant des chaînes en C10 à C13.

7. Procédé selon l'une quelconque des revendications 1 et 2, dans lequel le flux C14+ (120) comprend des paraffines en C16 à C18.
